# EUROPÄISCHE PATENTSCHRIFT

(11) **EP 3 646 812 B1**
(45) Veröffentlichungstag und Bekanntmachung des Hinweises auf die Patenterteilung: **06.01.2021**
(21) Anmeldenummer: 19400022.0
(22) Anmeldetag: 16.10.2019
(51) Int. Cl.: A61C 8/00

(54) **DENTALIMPLANTAT UND VERFAHREN ZU SEINER HERSTELLUNG**
DENTAL IMPLANT AND MANUFACTURING METHOD THEREOF
IMPLANT DENTAIRE ET SON PROCÉDÉ DE FABRICATION

(30) Priorität: 29.10.2018 DE 102018008528
(43) Veröffentlichungstag der Anmeldung: 06.05.2020
(73) Patentinhaber: ZM Präzisionsdentaltechnik GmbH, 18055 Rostock (DE)
(72) Erfinder: Milija, Mitrovic, 18055 Rostock (DE)
(74) Vertreter: Hansmann, Dierk

(56) Entgegenhaltungen:
- EP-A1- 2 742 905
- DE-A1-102011 015 299
- DE-U1-202006 011 844

## Beschreibung

Die Erfindung bezieht sich auf ein Dentalimplantat und einem Verfahren zur Herstellung, bestehend aus einem Verankerungselement aus keramischem Werkstoff, insbesondere auf Zirkoniumoxidbasis, wobei das Verankerungselement zervikal eine Sacklochbohrung aufweist, in das zur Bildung eines Hybridimplantats ein Einsatzelement aus einem Titanwerkstoff mit einem Innengewinde als Aufnahme für ein korrespondierendes Abutment als Zahnersatz über ein Gewinde einsetzbar und das Einsatzelement mittels Löten mit einem Glaslot im Verankerungselement festgesetzt ist.

Zweiteilige Dentalimplantate dieser Art als Hybridimplantate sind bereits in unterschiedlichen Ausbildungen, wie aus der DE 2011 015 299 A1 und der EP 26 88 510 B1, bekannt geworden. Auch aus der EP 27 42 905 A1 ist ein Dentalimplantat aus keramischem Werkstoff mit einer Bohrung zum Einsetzen einer Gewindehülse aus einem Titanwerkstoff und Befestigung durch Löten mit einem Glaslot bekannt. Ebenfalls ist aus der EP 20 39 319 A1 ein Dentalimplantat vorgeschlagen worden, das aus einem keramischen Werkstoff besteht und in einer Ausnehmung über ein Fügeverfahren ein separates Einsatzelement befestigbar ist. Weiterhin ist nach der WO 2012 126 447 A1 bekannt, ein Dentalimplantat aus einem keramischen Material an seiner Aussehfläche mit einer silikatischen Glaskeramik zu versehen und eine Schicht aus Titan aufzutragen.

Durch den Einsatz der unterschiedlichen Materialen mit dem Einsatzelement aus einem Titanwerkstoff für ein Abutment über korrespondierende Gewinde und dem Verankerungselement aus keramischem Werkstoff werden gegenüber einteiligen Dentalimplantaten die in der Praxis auftretenden Gewindeausrisse in dem keramischen Werkstoff des Verankerungselementes oder einen abrasierbarem Abtrag vermieden.

Eine Schwierigkeit bei Hybridimplantaten besteht darin, bei der Verbindung des Verankerungselementes aus keramischem Werkstoff und dem Einsatzelement aus Titanwerkstoff durch Löten mit Glaslot eine optimale Festigkeit und Spalt- sowie Blasenfreiheit der Verbindung zu erhalten.

Die Aufgabe der Erfindung ist es, durch eine entsprechende Ausbildung und ein einfaches Verfahren für eine reproduzierbare Lötverbindung mit Glaslot zwischen Verankerungselement aus keramischem Werkstoff und Einsatzelement aus Titanwerkstoff als homogene Lötung der Fügeflächen zu schaffen und eine Verbindung ohne Lufteinschlüsse zu gewährleisten.

Die Lösung dieser Aufgabe erfolgt erfindungsgemäss dadurch, dass das Einsatzelement mindestens im Fügebereich punktuelle Distanzhalter zur Bildung eines umlaufenden Spaltraumes aufweist und gegenüber dem Verankerungselement einen Überstand bildet sowie der Spaltraum mit einer Glaslotsuspension im Überschuss aufgefüllt und nach einer thermischen Lötphase der Überstand des Einsatzelementes und ein bestehender Lotüberschuss an der zervikalen Implantatseite abgetrennt sind.

Hierdurch wird auf einfache Weise über die Distanzhalter ein durchgehender Spaltraum gleichmässiger Breite geschaffen, um kritischen Spannungen in der Glasmatrix entgegen zu wirken. Ferner wird durch den Überstand des Verankerungselementes und der Überschuss der eingefüllten Glatlotsuspension Nachsaugematerial zur Verfügung gestellt, wobei auch ein Abfliessen in die Innengeometrie des Einsatzelementes vermieden wird. Der Überschuss an Glatlotsuspension ergibt ein Materialreservoir und vermeidet das Entstehen von Lunkern im Spaltraum auch unter Berücksichtigung von Schrumpfprozessen. Ein Kapillareffekt wird in Richtung der zervikalen Implantatseite konzentriert.

Um einen guten Materialfluss der Glaslotsuspension zu ermöglichen wird vorgeschlagen, dass das Einsatzelement im Bodenbereich zusätzlich Distanzhalter aufweist.

Ferner wird zur rissfreien Lötung vorgeschlagen, dass eine Breite des Spaltraumes nicht grösser als 0,5 µm ist.

Um die bei der Lötung entstehende dunkle Oxidschicht des Einsatzelementes aus Titanwerkstoff abzudecken wird vorgeschlagen, dass das Einsatzelement eine Vorbeschichtung mit keramischen Primer aufweist.

Für ein Verfahren zur Herstellung des Dentalimplantats wird vorgeschlagen, dass die Gaslotsuspension in die Bohrung des Verankerungselementes eingefüllt wird und das anschliessend eingesetzte Einsatzelement mit punktuellen Distanzhaltern die verdrängte Glaslotsuspension in einem umlaufenden Spaltraum aufnimmt, wobei das Einsatzelement einen Überstand zum Verankerungselement aufweist und die Glaslotsuspension einen Überschuss an der Eintrittsöffnung des Verankerungselementes bildet sowie der folgende Lötprozess unter Vakuum im Bereich von 800° C durchgeführt, wobei nach dem Lötprozess der Überstand des Einsatzelementes und des verbliebenen Glaslotes zur Bildung einer planen Oberfläche getrennt werden.

Hierdurch wird ermöglicht, ein Dentalimplantat nach der Aufgabenstellung mit den vorgenannten Vorteilen herzustellen.

Ferner wird vorgeschlagen, dass die Aussengeometrie des Einsatzelementes mit der Glaslotsuspension vor dem Einsetzen in das Verankerungselement zu benetzen ist.

Um eine Verdichtung und gute Verteilung der eingebrachten Glaslotsuspension zu erreichen ist vorgesehen, dass das Einsatzelement in seiner eingesetzten Position durch Vibration beaufschlagt wird.

Um ein Fliessen der Glaslotsuspension unter Druck und Vibration ohne einen Rheopexie-Effekt zu erreichen wird vorgeschlagen, dass die eingebrachte Glaslotsuspension eine Körnungsgrösse unterhalb von 15 µm aufweist.

In der Zeichnung ist ein Ausführungsbeispiel der Erfindung schematisch in vergrössertem Massstab dargestellt. Es zeigen:

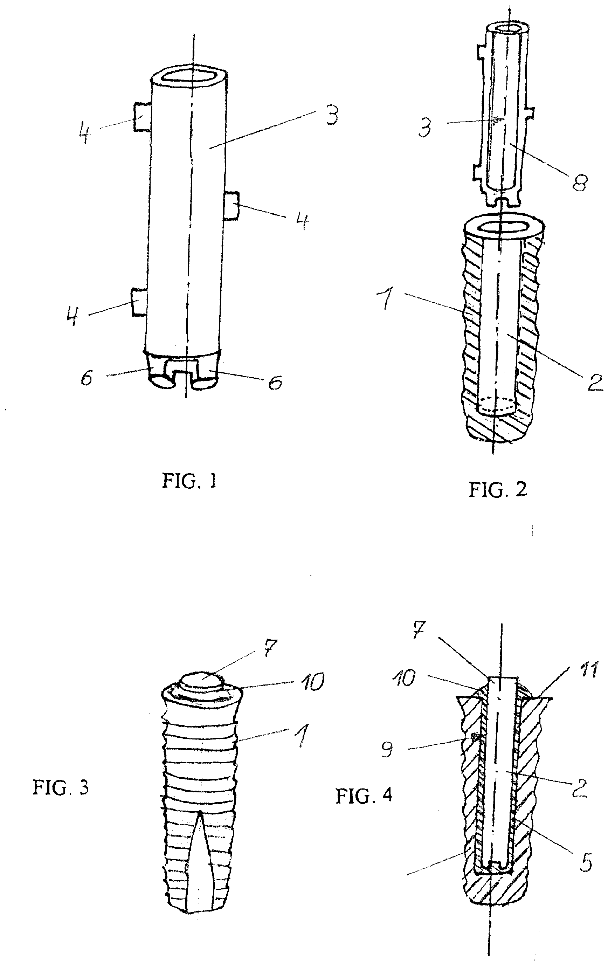

- Fig. 1: ein Einsatzelement des Dentalimplantats mit punktuellen Distanzhaltern,
- Fig. 2: eine schaubildliche Darstellung eines Verankerungselementes mit zugeordnetem Einsatzelement,
- Fig. 3: eine perspektivischen Darstellung eines Dentalimplantats mit Verankerungselement und aufgenommenem Einsatzelement sowie Überschuss der Glatlotsuspension auf der Implantatschulter und
- Fig. 4: eine Schnittdarstellung gemäss Fig. 3.

Zur Bildung eines Dentalimplantats als Hybridimplantat durch eine Lötverbindung mit Glaslot ist ein Verankerungselement aus einer Keramik auf Zirkoniumoxidbasis angeordnet, das ein Sackloch 2 aufweist. In das Sackloch 2 des Verankerungselementes 1 ist ein Einsatzelement 3 aus einem Titanwerkstoff einsetzbar, das am Umfang punktuelle Distanzhalter 4 aufweist und zur Bildung eines umlaufenden konstanten Spaltraumes 5 aufweist und das Einsatzelement 3 zentriert gehalten wird. In diesem Ausführungsbeispiel sind auch im Bodenbereich des Einsatzelementes 3 Distanzhalter 6 angeordnet. Das Einsatzelement 3 besitzt dabei in der eingesetzten Position gegenüber dem Verankerungselement 1 einen Überstand 7.

In bekannter Weise ist der Innenraum 8 der Aufnahme des Einsatzelementes 3 mit einem Innengewinde versehen, um ein nicht dargestelltes korrespondierendes Abutment als Zahnersatz über einen Gewindestift aufzunehmen.

Das Einsatzelement 3 kann in der Fügephase durch Löten auch aus einem Vollmaterial gebildet sein, wobei nach dem Lötvorgang die Aufnahme 8 mit dem Innengewinde eingebracht wird.

Vor einem Lötvorgang wird das Sackloch 2 im Verankerungselement 1 mit einer Glaslotsuspension 9 gefüllt und anschliessend das Einsatzelement 3 eingesetzt, wobei eine Bemessung derart erfolgt, dass ein Überschuss 10 der Glaslotsuspension 9 auf der Implantatschulter 11 und dem Überstand 7 des Einsatzelementes 3 gebildet wird.

Diese zusammengesetzte Anordnung wird einem Lötprozess im Bereich bis zu 800° C unter Vakuum unterzogen und anschliessend ein bestehender Lötüberschuss und der Überstand 7 des Einsatzelementes 3 abgetrennt.

Das Verfahren zur Herstellung des Dentalimplants wird somit im wesentlichen durch folgende Verfahrensschritte durchgeführt.

Das Verankerungselement 1 wird mit einer Glaslotsuspension befüllt und die Aussengeometrie des einzusetzenden Einsatzelement 3 mit der Glaslotsuspension benetzt. Dann wird das Einsatzelement 3 in das befüllte Verankerungselement 1 eingesetzt. Durch die Distanzhalter 4 wird das Einsatzelement 3 in dem Verankerungselement 1 unter Ausbildung eines konstanten Spaltraumes 5 zentriert angeordnet. Durch aufgebrachte Vibration wird die eingebrachte Glaslotsuspension 9 verdichtet und gleichmässig verteilt. Dabei ist die Glaslotsuspension derart bemessen, dass sie im oberen Bereich auf der Implantatschulter 11 als Überschuss 10 herausquillt, wobei der Überstand 7 des Einsatzelementes 3 eine Begrenzung bildet. Der benötigte Überschuss 10 kann somit nicht in die Innengeometrie des Einsatzelementes 3 fliessen, und es wird ein kontrollierter Raum für ein benötigtes Nachsaugmaterial geschaffen.

Beim thermischen Brand als Lötvorgang im Bereich bis zu 800° C unter Vakuum schrumpft die Glaslotsuspension und es besteht durch den Überschuss 10 Nachsaugmaterial. Dieses dient zur Vermeidung von Nachsauglunkern. Nach Beendigung des thermischen Brennvorganges wird der Überstand 7 des Einsatzelementes 3 und der bestehende Lötüberschuss 10 abgetrennt, so dass eine plane Oberfläche des Hybridimplantats entsteht.

## Patentansprüche

1. Dentalimplantat, bestehend aus einem Verankerungselement aus keramischem Werkstoff, insbesondere auf Zirkoniumoxidbasis, wobei das Verankerungselement (1) zervikal eine Sacklochbohrung (2) aufweist, in das zur Bildung eines Hybridimplantats ein Einsatzelement (3) aus einem Titanwerkstoff mit einem Innengewinde als Aufnahme für ein korrespondierendes Abutment als Zahnersatz über ein Gewinde einsetzbar und das Einsatzelement (3) mittels Löten mit einem Glaslot im Verankerungselement (1) festgesetzt ist, **dadurch gekennzeichnet, dass** das Einsatzelement (3) mindestens im Fügebereich punktuelle Distanzhalter (4) zur Bildung eines umlaufenden Spaltraumes (5) aufweist und gegenüber dem Verankerungselement (1) einen Überstand (7) bildet sowie der Spaltraum (5) mit einer Glaslotsuspension (9) im Überschuss (10) aufgefüllt und nach einer thermischen Lötphase der Überstand (7) des Einsatzelementes (3) und ein bestehender Lotüberschuss(10) an der zervikalen Implantatseite abgetrennt sind.

2. Dentalimplantat nach Anspruch 1, **dadurch gekennzeichnet, dass** das Einsatzelement (3) im Bodenbereich zusätzlich Distanzhalter (6) aufweist.

3. Dentalimplantat nach einem Anspruch 1 oder 2, **dadurch gekennzeichnet, dass** eine Breite des Spaltraumes (5) nicht grösser als 0,5 mm ist.

4. Dentalimplantat nach einem der Ansprüche 1 bis 3, **dadurch gekennzeichnet, dass** das Einsatzelement (3) eine Vorbeschichtung mit keramischen Primer aufweist.

5. Verfahren zur Herstellung eines Dentalimplantats nach Anspruch 1 bestehend aus einem Verankerungselement (1) aus keramischem Werkstoff, insbesondere auf Zirkoniumoxidbasis, wobei das Verankerungselement (1) zervikal eine Sacklochbohrung (2) aufweist, in das zur Bildung eines Hybridimplantats ein Einsatzelement (3) aus einem Titanwerkstoff mit einem Innengewinde als Aufnahme für ein korrespondierendes Abutment als Zahnersatz über ein Gewinde eingesetzt und das Verankerungselement (1) mit dem Einsatzelement (3) durch Löten mit einem Glaslot verbunden wird, **dadurch gekennzeichnet, dass** eine Gaslotsuspension in die Bohrung (2) des Verankerungselementes (1) eingefüllt wird und das anschliessend eingesetzte Einsatzelement (3) mit punktuellen Distanzhaltern (4) die verdrängte Glaslotsuspension (9) in einem umlaufenden Spaltraum (5) aufnimmt, wobei das Einsatzelement (3) einen Überstand (7) zum Verankerungselement (1) aufweist und die Glaslotsuspension (9) einen Überschuss (10) an der Eintrittsöffnung des Verankerungselementes (1) bildet sowie der folgende Lötprozess unter Vakuum im Bereich von 800° C durchgeführt,
wobei nach dem Lötprozess der Überstand (7) des Einsatzelementes (3) und des verbliebenen Glaslotes zur Bildung einer planen Oberfläche getrennt werden.

6. Verfahren nach Anspruch 5, **dadurch gekennzeichnet, dass** die Aussengeometrie des Einsatzelementes (3) mit der Glaslotsuspension (9) vor dem Einsetzen in das Verankerungselement (1) zu benetzen ist.

7. Verfahren nach einem der Ansprüche 5 oder 6, **dadurch gekennzeichnet, dass** das Einsatzelement (3) in seiner eingesetzten Position durch Vibration beaufschlagt wird.

8. Verfahren nach einem der Ansprüche 5 bis 7, **dadurch gekennzeichnet, dass** die eingebrachte Glaslotsuspension (9) eine Körnungsgrösse unterhalb von 15 µm aufweist.

## Claims

1. A dental implant consisting of an anchoring element made of ceramic material, in particular based on zirconium oxide, the anchoring element (1) having, from a cervical end, a blind hole (2) into which, in order to form a hybrid implant, an insert element (3) made of a titanium material and having an internal thread is insertable via a thread as receptacle for a corresponding abutment as dental prosthesis, the insert element (3) being fixed in the anchoring element (1) by means of soldering with a glass solder, **characterised in that** the insert element (3) has, at least in the joining region, spacers (4) at individual points for forming a circumferential gap (5) and forms a protrusion (7) relative to the anchoring element (1) and fills the gap (5) with a glass solder suspension (9) to excess (10) and, after a thermal soldering phase, the protrusion (7) of the insert element (3) and any solder excess (10) are cut off on the cervical implant side.

2. The dental implant according to claim 1, **characterised in that** the insert element (3) additionally has spacers (6) in the base region.

3. The dental implant according to claim 1 or 2, **characterised in that** the width of the gap (5) is not greater than 0.5 mm.

4. The dental implant according to one of claims 1 to 3, **characterised in that** the insert element (3) has a pre-coating with ceramic primer.

5. A method for producing a dental implant according to claim 1 consisting of an anchoring element (1) made of ceramic material, in particular based on zirconium oxide, the anchoring element (1) having, from a cervical end, a blind bore (2), into which, in order to form a hybrid implant, an insert element (3) formed from a titanium material and having an internal thread is inserted via a thread as receptacle for a corresponding abutment as dental prosthesis, and the anchoring element (1) being connected to the insert element (3) by soldering with a glass solder, **characterised in that** a glass solder suspension is filled into the bore (2) of the anchoring element (1) and the insert element (3) then inserted with spacers (4) at individual points receives the displaced glass solder suspension (9) in a circumferential gap (5), the insert element (3) having a protrusion (7) relative to the anchoring element (1) and the glass solder suspension (9) forming an excess (10) at the entry opening of the anchoring element (1), and the following soldering process being performed under vacuum in the region of 800 °C,
the protrusion (7) of the insert element (3) and of the remaining glass solder, after the soldering process, being cut off to form a flat surface.

6. The method according to claim 5, **characterised in that** the external geometry of the insert element (3) with the glass solder suspension (9) should be wetted prior to the insertion into the anchoring element (1).

7. The method according to one of claims 5 or 6, **characterised in that** the insert element (3) is exposed to vibration in its inserted position.

8. The method according to one of claims 5 to 7, **characterised in that** the introduced glass solder suspension (9) has a grain size below 15 µm.

## Revendications

1. Implant dentaire, composé d'un élément d'ancrage en matériau céramique, en particulier à base d'oxyde de zirconium, dans lequel l'élément d'ancrage (1) présente un perçage de trou borgne (2) sur le côté cervical, dans lequel, pour former un implant hybride, un élément d'insert (3) en matériau de titane avec un filetage femelle en tant que réception pour un pilier correspondant peut être inséré par le biais d'un filetage en tant que substitut de dent, et l'élément d'insert (3) est fixé dans l'élément d'ancrage (1) par brasage au verre, **caractérisé en ce que** l'élément d'insert (3) présente, au moins dans la zone de jointure, des écarteurs (4) ponctuels pour former un espace (5) circulaire et forme une saillie (7) face à l'élément d'ancrage (1), et l'espace (5) est rempli d'une suspension de brasage au verre (9) dans l'excédent (10) et après une phase de brasage thermique, la saillie (7) de l'élément d'insert (3) et un excédent de brasage (10) restant sont séparés sur le côté cervical de l'implant.

2. Implant dentaire selon la revendication 1, **caractérisé en ce que** l'élément d'insert (3) présente en plus des écarteurs (6) sur le fond.

3. Implant dentaire selon la revendication 1 ou 2, **caractérisé en ce qu'**une largeur de l'espace (5) n'est pas supérieure à 0,5 mm.

4. Implant dentaire selon l'une des revendications 1 à 3, **caractérisé en ce que** l'élément d'insert (3) présente un pré-revêtement avec un apprêt céramique.

5. Procédé de fabrication d'un implant dentaire selon la revendication 1, composé d'un élément d'ancrage (1) en matériau céramique, en particulier à base d'oxyde de zirconium, dans lequel l'élément d'ancrage (1) présente un perçage de trou borgne (2) sur le côté cervical, dans lequel, pour former un implant hybride, un élément d'insert (3) en matériau de titane avec un filetage femelle en tant que réception pour un pilier correspondant peut être inséré par le biais d'un filetage en tant que substitut de dent, et l'élément d'insert (3) est relié à l'élément d'ancrage (1) par brasage au verre, **caractérisé en ce qu'**une suspension de brasage au verre (9) est remplie dans le perçage (2) de l'élément d'ancrage (1) et l'élément d'ancrage (3) avec des écarteurs (4) ponctuels inséré ensuite reçoit la suspension de brasage au verre (9) poussée dans un espace circulaire (5), dans lequel l'élément d'ancrage (3) présente une saillie (7) vers l'élément d'ancrage (1) et la suspension de brasage au verre (9) forme un excédent (10) sur l'ouverture d'entrée de l'élément d'ancrage (1), et le procédé de brasage suivant est exécuté sous vide à 800 °C,
dans lequel après le procédé de brasage, la saillie (7) de l'élément d'ancrage (3) et du brasage au verre restant sont séparés pour former une surface plane.

6. Procédé selon la revendication 5, **caractérisé en ce que** la géométrie extérieure de l'élément d'insert (3) doit être mouillée avec la suspension de brasage au verre (9) avant l'insertion dans l'élément d'ancrage (1) .

7. Procédé selon la revendication 5 ou 6, **caractérisé en ce que** l'élément d'insert (3) reçoit une vibration dans sa position insérée.

8. Procédé selon l'une des revendications 5 à 7, **caractérisé en ce que** la suspension de brasage au verre (9) insérée présente une grosseur de grain inférieure à 15 µm.
